(19) Europäisches Patentamt
European Patent Office
Office européen des brevets

(11) Veröffentlichungsnummer: **0 036 076**
**B1**

(12) # EUROPÄISCHE PATENTSCHRIFT

(45) Veröffentlichungstag der Patentschrift: **22.02.84**

(51) Int. Cl.³: **C 07 D 233/40,** D 06 M 15/54

(21) Anmeldenummer: **81100957.0**

(22) Anmeldetag: **11.02.81**

(54) **Verfahren zur Herstellung von formaldehydfreien Ausrüstungsmitteln für cellulosehaltige Textilien und deren Verwendung.**

(30) Priorität: **18.03.80 US 131409**

(43) Veröffentlichungstag der Anmeldung: **23.09.81 Patentblatt 81/38**

(45) Bekanntmachung des Hinweises auf die Patenterteilung: **22.02.84 Patentblatt 84/8**

(84) Benannte Vertragsstaaten: **AT CH DE GB IT LI SE**

(56) Entgegenhaltungen:
**DE - B - 1 171 437**
**DE - B - 1 172 265**
**FR - A - 1 316 792**

(73) Patentinhaber: **BASF Aktiengesellschaft, Carl-Bosch-Strasse 38, D-6700 Ludwigshafen (DE)**

(72) Erfinder: **Petersen, Harro, Dr., Kalmitstrasse 23, D-6710 Frankenthal (DE)**
Erfinder: **Klippel, Friedrich, Stockholmer Weg 7, D-6700 Ludwigshafen (DE)**
Erfinder: **Pai, Panemangalore Subraya, 4100 Bridgewood Lane, Charlotte, N.C. 28211 (US)**
Erfinder: **Reinert, Friedrich, Dr., In der Dreispitz 11, D-6706 Wachenheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

# Verfahren zur Herstellung von formaldehydfreien Ausrüstungsmitteln für cellulosehaltige Textilien und deren Verwendung

Mit »Ausrüsten« ist das Krumpf- und Knitterfest-Ausrüsten — auch »Pflegeleicht-Ausrüsten« genannt — gemeint. Dieses erfolgt üblicherweise mit Aminoplastbildnern, d. h. Produkten, die am Stickstoff Hydroxymethyl- oder Alkoxymethylgruppen enthalten, wie vor allem hydroxymethylierten oder alkoxymethylierten Harnstoffen, cyclischen Harnstoffen, Carbamaten oder Aminotriazinen. Beim »Härten« oder »Fixieren« reagieren die N-Hydroxymethyl- oder N-Alkoxymethylgruppen unter dem Einfluß von Hitze und Katalysatoren untereinander und mit den Hydroxylgruppen der Cellulose unter Acetalbildung und Vernetzung. Dabei läßt es sich kaum vermeiden, daß die mechanische Festigkeit der Cellulose erheblich sinkt. Das ist ein gravierender Nachteil der Textilausrüstung. Ein weiterer, sehr schwerwiegender Nachteil bei der Ausrüstung mit diesen Produkten ist die Abspaltung von Formaldehyd während der Applikation sowie die nachträgliche Formaldehydabspaltung auf den mit diesen Produkten ausgerüsteten Geweben.

Es hat nicht an Versuchen gefehlt, die Probleme einer formaldehydfreien Ausrüstung zu lösen. Hierbei muß beachtet werden, daß praktisch alle nach chemischen Gesichtspunkten geeigneten Verbindungen, nämlich die Aziridin-, Epoxy-, Chlorhydroxy-, Vinyl- und Acryl-Verbindungen sehr toxisch und daher für eine Textilveredlung nicht in Betracht zu ziehen sind. Darüber hinaus können mit diesen Verbindungen die gewünschten Ausrüstungseigenschaften nur teilweise erreicht werden.

Neuerdings wurden im Zusammenhang mit einer formaldehydfreien Ausrüstung auch die N,N'-Dimethylderivate des 4.5-Dihydroxyethylenharnstoffes

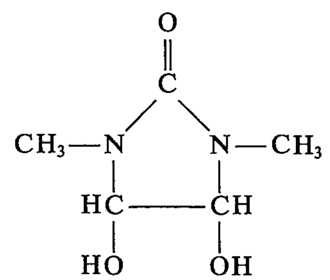

diskutiert und von einigen japanischen Chemikalienherstellern solche Produkte auch auf den Markt gebracht.

Frühere Arbeiten (S. L. Vail, Chem. and Ind., 1967, 305–309, sowie E. J. Gonzales, R. R. Benerito und R. J. Berni, Textile Research Journal 36 [1966], 365–371) zeigten, daß die mit N,N'-Dimethyl-4.5-dihydroxyethylenharnstoff erreichbaren Effekte eher denen des unbehandelten Materials als dem üblichen Standard einer hochveredelten Ware entsprechen.

Weiterhin sind beispielsweise in 4- und 5-Stellung durch niedere Alkylreste veretherte Dihydroxyethylenharnstoffe bekannt. Die Veretherung wird in alkoholischer Lösung, gegebenenfalls in Dioxan oder Tetrahydrofuran als Lösungsmittel, in Gegenwart von stark sauren Katalysatoren, beispielsweise sauren Ionenaustauschern, wie sulfoniertes Polystyrol (vgl. DE-AS 1 171 437 oder 1 172 265), oder starken Säuren bei einem pH-Wert von kleiner 4 (vgl. FR-PS 1 316 792) durchgeführt. Dabei sind Methylolverbindungen nicht ausgeschlossen.

Die Forderung nach formaldehydfreier Ausrüstung verbietet es, N-Hydroxymethylverbindungen einzusetzen. Aus toxikologischen Gründen können auch nicht die genannten Aziridin-, Epoxy-, Chlorhydroxy- sowie die Vinyl- und Acryl-Verbindungen verwendet werden.

Der Erfindung lag daher die Aufgabe zugrunde, ein einfaches und toxikologisch unbedenkliches Verfahren zur Herstellung und Verwendung eines formaldehydfreien Ausrüstungsmittels zu finden, das ausreichende Reaktivität mit hoher Lagerstabilität, ausreichender Ergiebigkeit und hoher Stabilität der daraus hergestellten Ausrüstungsflotten verbindet und außerdem ausreichend hydrolysenbeständig ist, um dem ausgerüsteten Textilgut eine weitgehend waschpermanente Krumpf- und Knitterfreiheit zu vermitteln. Darüber hinaus sollten die Reiß- und Scheuerfestigkeitsverluste nicht höher, nach Möglichkeit sogar geringer als bei Anwendung üblicher Ausrüstungsmittel sein, und der textile Griff sollte durch die Ausrüstung möglichst nicht ungünstig beeinflußt werden.

Es wurde nun ein Verfahren zur Herstellung von flüssigen, lagerbeständigen, formaldehydfreien Pflegeleicht-Ausrüstungsmitteln für cellulosehaltige Textilien auf der Basis von Harnstoff oder N,N'-disubstituierten Harnstoffen und Glyoxal gefunden, das allen diesen Anforderungen gerecht wird und in den Ansprüchen 1 und 2 definiert ist, wie folgt:

1. Verfahren zur Herstellung von lagerbeständigen, formaldehydfreien Pflegeleicht-Ausrüstungsmitteln für cellulosehaltige Textilien auf der Basis von Harnstoff oder N,N'-disubstituierten Harnstoffen und Glyoxal, dadurch gekennzeichnet, daß man in einer ersten Verfahrensstufe 1 bis 1,1 Mol Harnstoff oder einen symmetrisch disubstituierten Harnstoff der allgemeinen Formel I

$R^1-HNCONH-R^2$ I

in der $R^1$ und $R^2$ ein Wasserstoffatom, einen Alkylrest mit 1 bis 4 C-Atomen, Hydroxyethyl, Methoxyethyl oder Methoxypropyl bedeuten, mindestens drei Stunden in wäßriger Lösung zusammen mit einem Mol Glyoxal in Gegenwart von Puffergemischen bei pH-Werten zwischen 4 und 6,8 und Temperaturen von 20 bis 60° C hält und die erhaltene Lösung in einer zweiten Stufe mit mindestens 0,5 Mol eines Alkohols $R^4OH$, wobei $R^4$ für Methyl, Ethyl, Methoxyethyl, Hydroxyethyl, Methoxyethoxyethyl oder für den Rest eines Polyethylenglykols mit bis zu 10 Ethylenoxideinheiten steht, zu einer Verbindung der allgemeinen Formel II

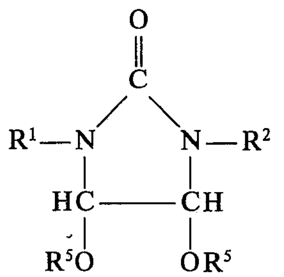

(II)

in der $R^1$ und $R^2$ die für Formel I angegebenen Bedeutungen haben und $R^5$ Wasserstoffatome bedeuten, die zu mindestens 25% durch einen Rest $R^4$ mit den obengenannten Bedeutungen ersetzt sind, umsetzt, indem man unter Zusatz einer Säure 1 bis 4 Stunden bei pH-Werten zwischen 2 und 4,5 und Temperaturen von 30 bis 65° C hält und schließlich den pH-Wert auf 4 bis 7 einstellt.

2. Verfahren zur formaldehydfreien Pflegeleichtausrüstung von cellulosehaltigen oder daraus bestehenden Textilien durch Imprägnieren derselben mit einer wäßrigen Lösung, enthaltend 2,5 bis 10 Gewichtsprozent, gerechnet als Festsubstanz, eines formaldehydfreien, vernetzbaren Ausrüstungsmittels und 0,6 bis 4 Gewichtsprozent eines sauren oder potentiell sauren Katalysators und Fixieren durch Erhitzen für 10 sec bis 15 min auf 100 bis 230° C, wobei das gemäß Anspruch 1 erhaltene Ausrüstungsmittel eingesetzt wird.

Die Umsetzung erfolgt in 2 Stufen, und zwar zunächst ohne den Alkohol $R^4OH$ während mindestens 3, vorzugsweise 4 bis 5 Stunden, bei pH 4 bis 6,8, vorzugsweise 5 bis 6,5, und bei Temperaturen von 20 bis 60, vorzugsweise 30 bis 40° C, dann nach Zusatz des Alkohols während 1 bis 4, vorzugsweise 2 bis 3 Stunden bei pH 2 bis 4,5, vorzugsweise 2,5, bis 4, und Temperaturen von 30 bis 65, vorzugsweise 40 bis 55° C. Schließlich wird ein pH-Wert im Bereich von 4 bis 7, vorzugsweise 4,5 bis 6,5, eingestellt. In den ersten 2 Stunden der 1. Stufe sollten 45° C nicht überschritten werden.

Als symmetrisch disubstituierte Harnstoffe können beispielsweise folgende Verbindungen eingesetzt werden: N,N'-Dimethylharnstoff, N,N'-Diethylharnstoff, N,N'-Diisopropylharnstoff, N,N'-Di-n-butylharnstoff, N-Methyl-N'-ethylharnstoff, N,N'-Di-hydroxyethylharnstoff, N,N'-Di-methoxyethylharnstoff, N,N'-Di-methoxypropylharnstoff.

Geeignete Alkohole $R^4OH$ sind, wie bereits vorstehend definiert, Methanol, Ethanol, Methylglykol, Ethylenglykol, Methyldiglykol und Polyethylenglykole mit bis zu 10 Ethylenoxideinheiten. Da die Hydroxylgruppen in 4- und 5-Stellung am cyclischen Harnstoff II zu mindestens 25% verethert sein sollen, ist pro Mol Harnstoff mindestens 0,5 Mol, vorzugsweise 1 bis 2 Mol Alkohol $R^4OH$ einzusetzen. Eine feste Obergrenze für die Alkoholmenge gibt es nicht, doch ist es wenig sinnvoll, wesentlich mehr als 2 Mol pro Mol Harnstoff einzusetzen, weil die Lösung dadurch nur unnötig verdünnt würde.

Als Puffergemische lassen sich folgende Verbindungen verwenden: Alkaliacetate/Essigsäure, Alkalitartrate/Weinsäure, Alkalicitrate/Citronensäure, Alkaliphthalate/Phthalsäure, Alkaliglykolate/Glykolsäure sowie deren Mischungen. Allgemein handelt es sich, ebenso wie hier, bei Puffergemischen bekanntlich um Mischungen von Salzen schwacher Säuren mit beliebigen Säuren. Ihre Einsatzmengen sind stets so zu wählen, daß unter den zu erwartenden Bedingungen die Einhaltung des gewünschten pH-Bereiches (hier 4 bis 6,8, vorzugsweise 5 bis 6,5) gewährleistet bleibt. Für das erfindungsgemäße Verfahren können schon 2 Gewichtsprozent, bezogen auf den als Ausgangsverbindung eingesetzten Harnstoff, an einem Salz einer schwachen Säure, wie Natriumacetat, genügen. In der Regel werden 5 bis 15% eingesetzt. Nach oben gibt es keine Mengengrenze, doch ist es wenig sinnvoll, mehr als 20 Gew.-% einzusetzen.

Bei der üblichen Herstellung von N,N'-Dimethyl-4.5-dihydroxyethylenharnstoff wird fester symmetrischer Dimethylharnstoff mit der wäßrigen Lösung von Glyoxal (z. B. 40%ig) im Molverhältnis 1 : 1 im schwach sauren oder schwach alkalischen Bereich bei Temperaturen zwischen 20 und 60° C umgesetzt. Die Cyclisierung zum N,N'-Dimethyl-4.5-dihydroxyethylenharnstoff verläuft relativ langsam und beansprucht für einen Umsatz von mehr als 90% Reaktionszeiten bis zu 2 Tagen. Die Reaktion erfordert zur Bindung des gesamten Glyoxals unter diesen Reaktionsbedingungen mehrere

Wochen. Eine Erhöhung der Temperatur bedingt eine starke Verfärbung des Produktes. Nicht umgesetzte Restmengen des Glyoxals führen zu einer starken Vergilbung bis Bräunung bei der Ausrüstung der Gewebe. Es ist zwar möglich, den im Reaktionsgemisch enthaltenen N,N'-Dimethyl-4.5-dihydroxyethylenharnstoff nach Abkühlen und Kristallisieren durch Filtration abzutrennen und gegebenenfalls durch Umkristallisieren zu reinigen. Diese Maßnahmen sind jedoch mit erheblichen Ausbeuteverlusten verbunden. Abgesehen davon sind die rohen und ebenso die gereinigten Lösungen von N,N'-Dimethyl-4.5-dihydroxyethylenharnstoff als Ausrüstungsmittel für cellulosehaltige Textilien wenig geeignet, wie aus dem Vergleichsversuch 2 hervorgeht. Vergleichsversuch 3 zeigt, daß auch die Veretherung, genauer: Acetalisierung der OH-Gruppen in den Stellungen 4 und 5 mit Alkoholen nur zu einer teilweisen Verminderung der Vergilbung der Gewebe bei der Ausrüstung führt. Weitere Untersuchungen haben gezeigt, daß die 4,5-Dihydroxyethylenharnstoffe unter der Einwirkung von Säuren, beispielsweise von latent sauren Katalysatoren, während der Hochveredlung in Hydantoine und andere stark gefärbte Nebenprodukte umgewandelt werden (H. Petersen, Textilveredlung 3 [1968], 51 – 62). Außerdem können die 4.5-Dihydroxyethylenharnstoffe unter dem Einfluß von Säuren oder latent sauren Katalysatoren in der Hitze in Glyoxaldiureine unter Freisetzung von Glyoxal umgewandelt werden. Freigesetztes Glyoxal führt, wie erwähnt, ebenfalls zu einer Vergilbung der Gewebe.

Überraschenderweise kann man bei der erfindungsgemäßen Herstellung von mit Alkoholen teilacetalisierten 4.5-Dihydroxyethylenharnstoffen oder N,N'-disubstituierten 4.5-Dihydroxyethylenharnstoffen praktisch alle gewünschten Eigenschaften erreichen sowie die unerwünschten Nebenreaktionen vermeiden: In Gegenwart der Puffergemische wird die Umsetzung von Harnstoff oder symmetrisch disubstituierten Harnstoffen mit Glyoxal zu 4.5-Dihydroxyethylenharnstoffen oder N,N'-disubstituierten 4.5-Dihydroxyethylenharnstoffen so beschleunigt, daß die Cyclisierung bereits nach wenigen Stunden ohne Verfärbung der Reaktionslösung bei Temperaturen unter 50° C quantitativ abgeschlossen ist. Im Reaktionsgemisch ist kein freies Glyoxal mehr nachweisbar. Die Reaktion verläuft in Gegenwart der Puffersubstanzen ohne Bildung von Nebenprodukten und führt zu einer hundertprozentigen Ausbeute. Eine Reinigung ist also nicht mehr erforderlich. Die erhaltenen Reaktionsgemische werden zur Erzielung optimaler Eigenschaften mit Alkoholen in Gegenwart von Säuren teilacetalisiert und können direkt für die Textilhochveredlung eingesetzt werden. Neben diesen überraschenden Befunden (kurze Reaktionszeiten, quantitative Ausbeute, keine Nebenproduktbildung, kein Restglyoxal) wurde ein weiterer; sehr überraschender Effekt bei der Ausrüstung von cellulosehaltigen Geweben mit den gepufferten Reaktionsgemischen gefunden: Verwendet man bei der Hochveredlung von cellulosehaltigen Textilien unter den üblichen Applikationsbedingungen ungepufferte Lösungen von z. B. N,N'-Dimethyl-4.5-dihydroxyethylenharnstoff III oder N, N'-Dimethyl-4.5-dimethoxyethylenharnstoff IV, so werden keine annehmbaren Ausrüstungseffekte und bei Verwendung von III eine starke Vergilbung der Gewebe beobachtet, wie die Vergleichsversuche 2 und 3 zeigen.

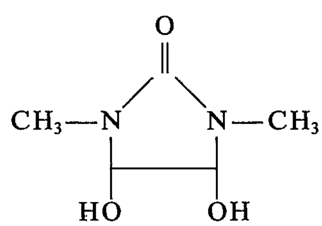

(III)

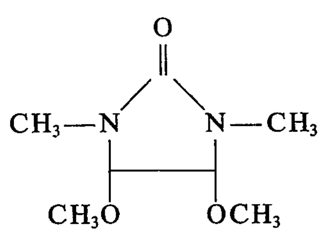

(IV)

Bei Einsatz der erfindungsgemäß erhältlichen Produkte, d. h., der unter Verwendung von Puffermischungen hergestellten Lösungen der Verbindungen II, werden auf Cellulose enthaltenden Geweben Ausrüstungseffekte mit geringen Krumpfwerten, hohen Monsanto-Noten und Durable-Press-Werten bei geringen Reiß- und Scheuerfestigkeitsverlusten und ohne Verfärbung erhalten. Der Griff der Gewebe bleibt weich. Die Benetzbarkeit und Feuchtigkeitsaufnahme wird im Vergleich zum

nicht ausgerüsteten Gewebe kaum beeinflußt.

Die Herstellung der erfindungsgemäß erhältlichen Produkte erfolgt zweckmäßig durch Umsetzung einer 40%igen wäßrigen Glyoxallösung mit pulverförmig zugesetztem Harnstoff oder N,N'-disubstituiertem Harnstoff, wobei die Lösung mit Schwefelsäure, Salzsäure oder einer a.. ⁻ren Säure auf pH 1—2 angesäuert und vor, unmittelbar nach oder gleichzeitig mit der Harnstoffzugabe mit einer solchen Menge eines Alkalisalzes einer organischen Säure, z. B. Natriumacetat oder Natriumcitrat od. dgl., versetzt wird, daß sich ein pH-Wert von 4—6,8, vorzugsweise 5—6,5, einstellt. Die Reaktionstemperatur wird zumindest während der ersten beiden Stunden unterhalb 50°C gehalten. Nach ca. 4—5stündigem Rühren zwischen 30 und 40°C ist die Reaktion beendet. Wie bereits erwähnt, hat sich als vorteilhaft erwiesen, die OH-Gruppen in den Stellungen 4 und 5 teilweise mit Alkoholen zu acetalisieren. Zu diesem Zweck wird zu der vorgehend beschriebenen Lösung z. B. Methanol oder ein anderer, oben unter der Bezeichnung $R^4OH$ genannter Alkohol zugesetzt, mit einer Mineralsäure auf einen pH-Wert von 2,5—4 eingestellt und 2—3 Stunden bei 40—55°C gerührt. Danach kann man das Gemisch mit Lauge oder einem Alkalisalz einer organischen Säure auf einen pH-Wert von 4,5—6,5 einstellen oder auch mit einem Puffersalz versetzen.

Die nach dem erfindungsgemäßen Verfahren hergestellten Ausrüstungsmittel sind lagerbeständig und verfärben sich nicht. Im Gegensatz dazu verfärben sich die wäßrigen Lösungen eines reinen N,N'-Dimethyl-4.5-dihydroxyethylenharnstoffes beim Lagern bereits nach einigen Wochen sehr stark.

Die Anwendung der erfindungsgemäß erhältlichen Ausrüstungsmittel erfolgt in an sich bekannter Weise, und zwar in Form eines wäßrigen Imprägnierbades, dem im allgemeinen die für die Vernetzung erforderlichen Katalysatoren zugesetzt werden, obwohl diese selbstverständlich im Prinzip auch in einem getrennten Arbeitsgang aufgebracht (z. B. fouladiert, gesprüht oder gepflatscht) werden könnten. Als Katalysatoren eignen sich besonders potentiell saure Salze, die für die Zwecke der Textilausrüstung allgemein bekannt und gebräuchlich sind. Als solche kommen beispielsweise Ammoniumsalze starker Säuren, Magnesiumchlorid, Zinkchlorid, Aluminiumchlorid, Zinknitrat, Salze von Fluoroboraten u. dgl. in Betracht. Auch Mischungen mehrerer Katalysatoren können verwendet werden. In vielen Fällen können mit Vorteil auch Mischungen dieser Katalysatoren mit hydroxylgruppenhaltigen organischen Säuren (z. B. Glykolsäure od. dgl.) eingesetzt werden. Die Katalysatormenge liegt üblicherweise im Bereich von 25 bis 45, vorzugsweise 30 bis 40 Gew.-%, bezogen auf den Festgehalt an Ausrüstungsmittel (Vernetzer).

Die Konzentration der Ausrüstungsflotte an Ausrünstungsmittel (gerechnet als Festkörper) richtet sich in üblicher Weise nach dem angestrebten Effekt. Sie liegt im allgemeinen zwischen 25 und 100 g/l. Das Behandlungsgut wird mit dem Imprägnierbad in üblicher Weise getränkt. Vorzugsweise bedient man sich dazu eines Foulards, das getränkte Gut befreit man in bekannter Weise, beispielsweise durch Abquetschen, von überschüssiger Imprägnierflüssigkeit. Der Faserauftrag liegt bei 2 bis 8, vorzugsweise 3 bis 6%. Der Auftrag der Ausrüstungsmittel kann auch nach einer MA-Technik (Minimalauftrag, siehe Triatex-Verfahren) oder über einen Minimalauftrag nach einer Schaumauftragstechnik erfolgen. Man kann das imprägnierte Fasergut mehr oder weniger trocknen und es dann in Gegenwart der sauren oder potentiell sauren Katalysatoren auf eine Temperatur zwischen 100 und 230°C, vorzugsweise auf 130 bis 210°C, erhitzen. Im allgemeinen ist unter diesen Bedingungen die Reaktion in 20 Sekunden bis 15 Minuten beendet. Einer höheren Temperatur entspricht selbstverständlich eine kürzere Zeit und umgekehrt. Man kann das Fasergut während des Trocknens oder danach mechanisch formen, beispielsweise durch Stauchen, Kräuseln, Bügeln, Kalandern, Prägen oder Plissieren. Auf diese Weise ausgerüstete, Cellulose enthaltende Textilien sind permanent knitterfest und krumpfecht. Hierzu kommen als weitere Vorteile ein relativ weicher Griff der ausgerüsteten Textilien, geringe Reißfestigkeitsverluste, geringe Scheuerfestigkeitsverluste, absolute Chlorechtheit und absolute Formaldehydfreiheit.

Zusammen mit den erfindungsgemäß einzusetzenden Ausrüstungsmitteln können auch die üblichen Hydrophobier-, Weichmachungs-, Egalisier-, Netz- und Appreturmittel sowie Griffvariatoren mitverwendet werden. Hydrophobiermittel sind z. B. aluminium- oder zirkonhaltige Paraffin-Wachs-Emulsionen sowie siliconhaltige Zubereitungen. Als Weichmachungsmittel seien Oxethylierungsprodukte von höheren Fettsäuren, Fettalkoholen oder Fettsäureamiden, höhermolekulare Polyglykolether, höhere Fettsäuren, Fettalkoholsulfonate, N-Stearylharnstoffverbindungen genannt. Als Egalisiermittel können wasserlösliche Salze von sauren Estern mehrbasischer Säuren mit Ethylenoxid- oder Propylenoxid-Addukten längerkettiger oxalkylierbarer basischer Grundstoffe verwendet werden. Netzmittel sind beispielsweise Salze der Alkylnaphthalinsulfonsäuren, die Alkalisalze des sulfonierten Bernsteinsäuredioctylesters und die Anlagerungsprodukte von Alkylenoxiden an Fettalkohole, Alkylphenole, Fettamine u. dgl. Als Appreturmittel kommen Celluloseether oder -ester und Alginate in Betracht, außerdem Lösungen oder Dispersionen synthetischer Polymerisate und Polykondensate, wie von Polyethylen, Polyamiden, oxethylierten Polyamiden, Polyvinylethern, Polyvinylalkoholen, Polyacrylsäure oder deren Estern und Amiden sowie von entsprechenden Polymethacrylverbindungen, Polyvinylpropionat, Polyvinylpyrrolidon, von Mischpolymerisaten, z. B. von solchen aus Vinylchlorid und Acrylsäureestern, aus Butadien und Styrol bzw. Acrylnitril oder aus 1.1-Dichlorethylen, β-Chloralkylacrylsäureestern oder Vinylethylether und Acrylsäureamid oder den Amiden der Crotonsäure oder Maleinsäure. Als Griffvariatoren kommen hier z. B. Polyvinylacetat und Polyacrylate,

gegebenenfalls in Mischung miteinander, in Betracht. Für absolut formaldehydfreie Ausrüstungen können selbstverständlich keine Hydroxymethyl- oder Alkoxymethylgruppen enthaltenden Ausrüstungs- und Hilfsmittel eingesetzt werden. Diese zusätzlichen Hilfsmittel werden im allgemeinen in Mengen von 0,3 bis 4%, vorzugsweise 1 bis 2,5%, bezogen auf das Gewicht des trockenen Textilgutes, angewendet. In besonderen Fällen kann man diese Mengen auch überschreiten.

In speziellen Fällen, z. B. für die Hochveredlung von reinen Baumwollgeweben mit der Forderung sehr hoher Monsanto-Glättebildnoten, kann man die erfindungsgemäß hergestellten Produkte mit besonderem Erfolg zusammen mit handelsüblichen vernetzenden Silicon-Elastomeren einsetzen.

Es lassen sich auch an Stelle von oder in Kombination mit den genannten Silicon-Elastomeren die in der DE-PS 2 334 655 beschriebenen Verbindungen der Formel V

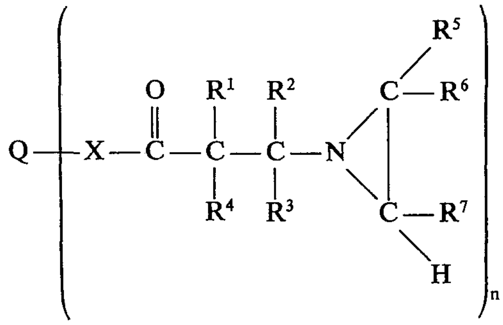

(V)

in der

| | |
|---|---|
| $R^1$ bis $R^7$ | Wasserstoffatome oder $C_{1-3}$-Alkylreste, |
| Q | den Rest eines n-wertigen Alkohols oder Phenols, |
| n | 2 oder 3, |
| X | eine Polyetherkette aus Butoxy- und/oder Propoxy- und gegebenenfalls Ethoxyeinheiten mit einem Atomverhältnis von C : O von mindestens 2,67 : 1 und einem Molekulargewicht von 150 bis 1500 bei n = 2, von 150 bis 3000 bei n = 3 bedeuten, |

mit gutem Erfolg einsetzen.

Als Cellulose enthaltende Textilien kommem Mischungen von Baumwolle oder Regeneratcellulose mit Synthesefasern, insbesondere mit Polyesterfasern, in Betracht.

Die in den folgenden Beispielen genannten Teile und Prozente sind Gewichtseinheiten.

## Beispiel 1

In einer Rührapparatur werden 308 Teile einer 40%igen Glyoxal-Lösung vorgelegt. Dazu werden 1,42 Teile Citronensäure-Monohydrat und 4,6 Teile Natriumacetat (wasserfrei) eingetragen. Der pH-Wert der Lösung liegt bei 4,6 bis 4,7. Unter Kühlung werden 135 Teile N,N'-Dimethylharnstoff zugegeben. Der pH-Wert stellt sich dabei auf 5,6 – 5,7 ein. Nach vierstündigem Rühren bei 30 – 35° C werden 69 Teile Methanol und 14,4 Teile Citronensäure-Monohydrat zugegeben und die Lösung weitere 2 Stunden bei 50° C gerührt. Der pH-Wert liegt dabei bei 4,2 bis 4,3. Dazu werden anschließend 10,4 Teile wasserfreies Natriumacetat gegeben und der pH-Wert mit verdünnter Natronlauge auf 5,6 – 5,7 eingestellt. Nach Verdünnen mit 81 Teilen Wasser wird eine 50%ige Vernetzerlösung erhalten, die gebrauchsfertig ist.

## Beispiel 2

In einer Rührapparatur werden 224 Teile einer 40%igen wäßrigen Glyoxallösung mit ca. 0,7 Teilen 75%iger Schwefelsäure auf pH 1,2 eingestellt. Dazu werden 146 Teile N,N'-Dimethylharnstoff zusammen mit 5 Teilen wasserfreien Natriumacetats innerhalb von 10 Minuten unter Rühren gegeben. Der pH-Wert liegt bei ca. 6. Unter Kühlen wird ca. 4 Stunden bei 40° C gerührt. Anschließend werden zum Cyclisierungsprodukt 74,5 Teile Methanol und 2 Teile 75%iger Schwefelsäure zugefügt. Hierbei stellt sich ein pH-Wert von ca. 3,9 ein. Nach zweistündigem Rühren bei 50°C werden 11,3 Teile wasserfreies Natriumacetat und 88,5 Teile Wasser zugesetzt. Mit ca. 5 Teilen 75%iger Schwefelsäure wird ein pH-Wert von ca. 4 eingestellt. Es werden 500 Teile einer 50%igen gebrauchsfertigen Vernetzerlsöung erhalten.

Beispiel 3

Die Mischung von 656 Teilen einer 40%igen Glyoxal-Lösung, 428 Teilen N,N'-Dimethylharnstoff und 14,7 Teilen wasserfreien Natriumacetats wird mit konzentrierter Salzsäure auf einen pH-Wert von 6,05 eingestellt und 4 Stunden bei 20 bis 30°C gerührt. Hierbei ist eine intensive Kühlung während der ersten 2 Stunden erforderlich. Anschließend werden 218 Teile Methanol und 12 Teile konz. Salzsäure zugegeben. Das Reaktionsgemsich weist einen pH-Wert von ca. 4 auf und wird 2 Stunden bei 50°C gerührt. Dazu werden 32,9 Teile wasserfreien Natriumacetats und 259 Teile Wasser gegeben. Der pH-Wert wird mit ca. 29 Teilen konz. Salzsäure auf einen Wert von ca. 4,2 eingestellt. Es werden 1600 Teile einer gebrauchsfertigen 50%igen Vernetzerlösung erhalten.

Beispiel 4

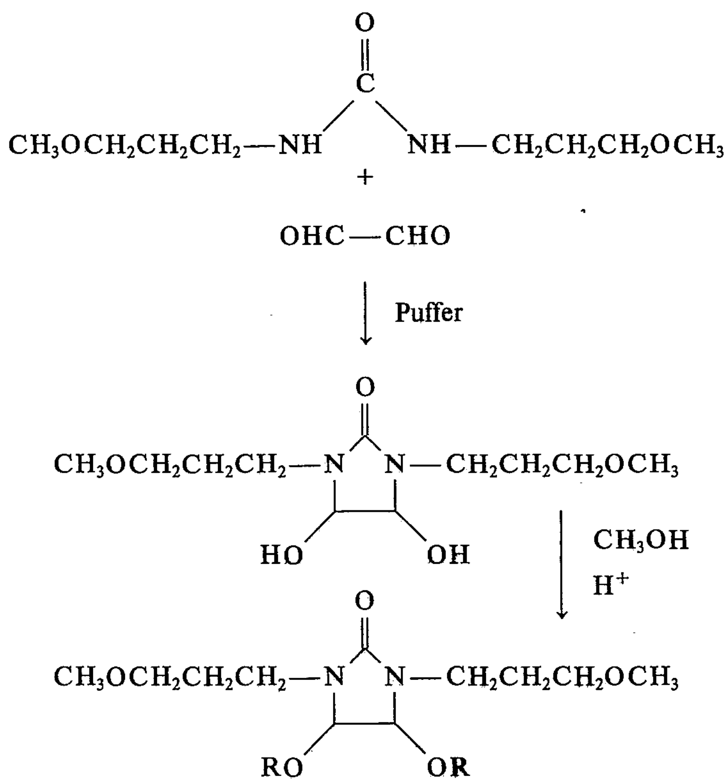


$R=H, CH_3$

In einer Rührapparatur werden 145 Teile einer 40%igen wäßrigen Glyoxal-Lösung mit 0,2 Teilen 75%iger Schwefelsäure versetzt. In dieses Gemisch werden unter Rühren 204 Teile N,N'-Bismethoxypropylharnstoff und 3,5 Teile wasserfreies Natriumacetat eingetragen. Der pH-Wert beträgt dann 4,3. Das Reaktionsgemisch wird 2 Stunden auf 45, dann 2 weitere Stunden auf 60°C erwärmt. Anschließend werden 60 Teile Methanol und 3 Teile 75%iger Schwefelsäure zugefügt. Hierbei stellt sich ein pH-Wert von ca. 2,8–3,0 ein. Nach zweistündigem Rühren bei 50°C werden 8 Teile Natriumacetat und 310 Teile Wasser zugefügt. Mit 75%iger Schwefelsäure wird ein pH-Wert von 5,0 bis 5,2 eingestellt. Es werden 630 Teile einer 50%igen Vernetzerlösung erhalten.

Beispiel 5

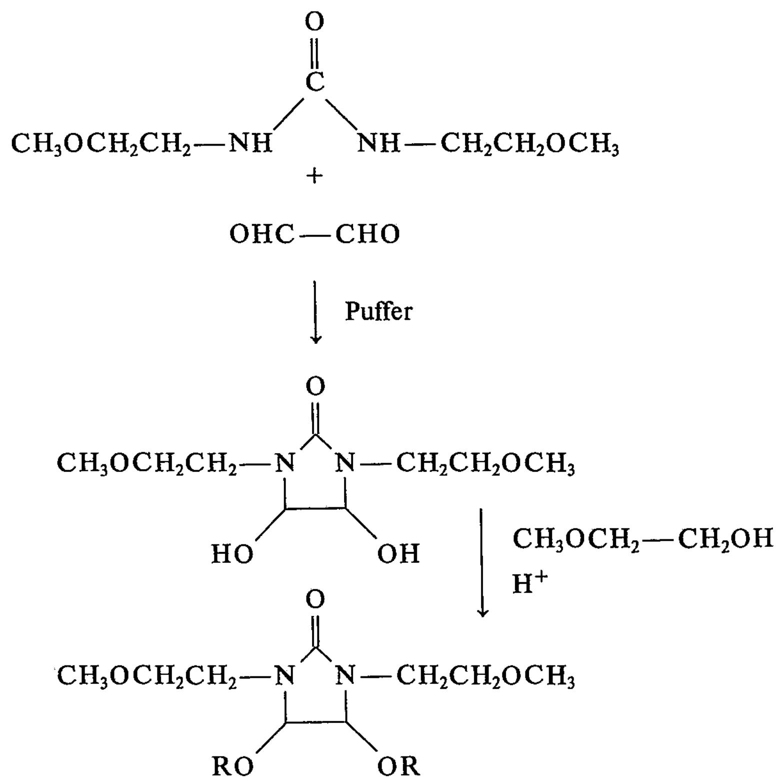


$R{=}H,\ CH_2CH_2OCH_3$

Das Gemisch von 176 Teilen N,N'-Bismethoxyethylharnstoff und 145 Teilen einer 40%igen Glyoxallösung wird mit 0,4 Teilen 75%iger Schwefelsäure und 4,5 Teilen Natriumacetat in einem Rührkolben 2 Stunden bei pH 4 auf 45, dann 2 weitere Stunden auf 60° C unter Rühren erwärmt. Anschließend werden 115 Teile Methylglykol und 3,5 Teile 75%iger Schwefelsäure hinzugefügt, so daß sich pH 2,1 einstellt. Das Reaktionsgemisch wird 2 Stunden auf 50° C erwärmt. Nach Zusatz von 340 Teilen Wasser wird mit 50%iger Natronlauge ein pH-Wert von ca. 5,5 eingestellt. Es werden 700 Teile einer ca. 50%igen Vernetzerlösung erhalten. Gegebenenfalls wird die Lösung mit Aktivkohle entfärbt und filtriert.

Beispiel 6

In einer Rührapparatur werden 1450 Teile einer 40%igen wäßrigen Glyoxallösung mit 15 Teilen Citronensäure-Monohydrat und 40 Teilen Natriumacetat versetzt. Unter Rühren werden hierzu 600 Teile Harnstoff eingetragen. Der pH-Wert liegt zwischen 4,3 und 4,5. Nach 5stündigem Rühren bei 35–40° C werden 600 Teile Methanol und 5 Teile 75%iger Schwefelsäure zugefügt, wobei sich ein pH-Wert von ca. 3,5 einstellt. Nach 2stündigem Rühren bei 50° C wird mit Natriumacetat ein pH-Wert von 4 eingestellt. Es werden 2700 Teile einer ca. 45%igen gebrauchsfertigen Vernetzerlösung erhalten.

Testergebnisse

Beispiel A

Das auszurüstende Material besteht aus 100%igem Baumwollnesselgewebe mit folgenden Eigenschaften:

| | |
|---|---|
| Naßknitterwinkel (Summe von Kette und Schuß = K + S) | 120° |
| Trockenknitterwinkel (K + S) | 85° |
| Monsanto-Bild (machine dry = md) nach einer Wäsche 20 min bei 60° C | 2 |
| Reißfestigkeit, Schuß | 470 N |

| | |
|---|---|
| Waschkrumpf nach einer Wäsche bei 60° C, | |
| Kette | 4,0% |
| Schuß | 3,0% |

Das Gewebe wird mit einer wäßrigen Lösung von 150 g/l nach Beispiel 1 hergestellter 50%iger Vernetzerlösung und 30 g/l Magnesiumchlorid-Hexahydrat als Katalysator mittels Foulard imprägniert. Die Flottenaufnahme beträgt ca. 70%. Getrocknet wird bei 110°C auf eine Restfeuchte von ca. 8%. Anschließend wird 4 min bei 150°C kondensiert. Das so ausgerüstete Gewebe liefert folgende Testergebnisse:

| | |
|---|---|
| Naßknitterwinkel (K+S) | 210° |
| Trockenknitterwinkel (K+S) | 198° |
| Monsanto-Bild, md, nach 1 × 20 min 60° C-Wäsche | 3,75 |
| Reißfestigkeit, Schuß | 360 N |
| Waschkrumpf nach 1 × 60° C-Wäsche, | |
| Kette | 1,0% |
| Schuß | 0,0% |
| Freie Formaldehydwerte nach LAW 112-1973 | 0 ppm |

## Beispiel B

Die in Beispiel A erwähnte Baumwollware wird mit folgender Lösung in üblicher Weise mittels Foulard imprägniert. Flottenaufnahme ca. 70%.

| | |
|---|---|
| 150 g/l | 50%ige Vernetzerlösung nach Beispiel 1 |
| 10 g/l | einer handelsüblichen 35%igen wäßrigen Emulsion eines hochmolekularen Aminosiloxans |
| 5 g/l | einer handelsüblichen 30%igen wäßrigen Emulsion eines relativ niedermolekularen Polysilxoans als Vernetzer |
| 3 g/l | Wasserstoffperoxid, 35%ig |
| 30 g/l | Magnesiumchlorid-Hexahydrat |

Trocknung und Kondensation entsprechen Beispiel A. Die anwendungstechnischen Prüfungen ergeben folgende Werte:

| | |
|---|---|
| Naßknitterwinkel (K+S) | 247° |
| Trockenknitterwinkel (K+S) | 266° |
| Monsanto-Bild, md, nach 1 × 20 min 60° C-Wäsche | 4 |
| Reißfestigkeit, Schuß | 320 N |
| Waschkrumpf nach 1 × 60° C-Wäsche, | |
| Kette | 0,0% |
| Schuß | 0,5% |
| Formaldehydwerte nach LAW 112-1973 | 0 ppm |

Außerdem wird neben einem sehr guten Warengriff eine deutliche Verbesserung der Vernähbarkeit erzielt.

## Beispiel C

Die in Beispiel A genannte Baumwollware wird mit folgender Lösung imprägniert und im Foulard auf ene Flottenaufnahme von ca. 70% abgequetscht.

| | |
|---|---|
| 150 g/l | 50%ige Vernetzerlösung nach Beispiel 1 |
| 30 g/l | einer Lösung von 20 g einer Mischung aus 50 Teilen Polytetrahydrofuran-2000-*bis-(β-aziridinopropionsäureester), 5 Teilen eines Additionsproduktes von 7 Mol Ethylenoxid an einem $C_{12-14}$-Alkohol und 45 Teilen Methanol in 10 g 1n Essigsäure |
| 30 g/l | Magnesiumchlorid-Hexahydrat |

* 2000 bezeichnet das mittlere Molgewicht der Polytetrahydrofuran-Kette.

Trocknung und Kondensation entsprechen Beispiel A. Die anwendungstechnischen Prüfungen ergeben folgende Werte:

| | |
|---|---|
| Naßknitterwinkel (K+S) | 236° |

| | |
|---|---|
| Trockenknitterwinkel (K+S) | 251° |
| Monsanto-Bild, md, nach einer Wäsche von 20 min bei 60° C | 4 |
| Reißfestigkeit, Schuß | 350 N |
| Waschkrumpf nach einer Wäsche von 20 min bei 60° C, | |
| Kette | 0,5% |
| Schuß | 0,5% |
| Formaldehyd nach LAW 112-1973 | 0 ppm |

Es wird ein seidenähnlicher, waschpermanenter Griff erhalten. Die Vernähbarkeit ist verbessert.

## Beispiel D

Die in Beispiel A erwähnte Baumwollware wird mit nachstehender Lösung bei einer Flottenaufnahme von 70% mittels Foulard imprägniert.

| | |
|---|---|
| 150 g/l | gemäß Beispiel 2 erhaltener, 50%iger Vernetzerlösung |
| 30 g/l | eines handelsüblichen Textilweichmachers auf der Basis eines Fettsäure-Kondensationsproduktes |
| 30 g/l | Magnesiumchlorid-Hexahydrat |

Trocknung und Kondensation wie in Beispiel A geschildert.

| | |
|---|---|
| Naßknitterwinkel (K+S) | 221° |
| Trockenknitterwinkel (K+S) | 210° |
| Monsanto-Bild, md, nach 1 x 20 min 60° C-Wäsche | 4 |
| Reißfestigkeit, Schuß | 350 N |
| Waschkrumpf nach 1 x 20 min 60° C-Wäsche, | |
| Kette | 0,0% |
| Schuß | 0,5% |
| Formaldehydwerte nach LAW 112-1973 | 0 ppm |

## Beispiel E

Die in Beispiel A erwähnte Baumwollware wird mit folgender Ausrüstungsflotte standardmäßig imprägniert. Die Flottenaufnahme beträgt ca. 70%. Trocknung und Kondensation wie in Beispiel A geschildert.

| | |
|---|---|
| 150 g/l | der nach Beispiel 3 erhaltenen 50%igen Vernetzerlösung |
| 30 g/l | eines handelsüblichen Textilweichmachers auf Basis eines oxäthylierten $C_{16-18}$-Alkohols |
| 20 g/l | eines handelsüblichen Griffvariators auf Basis einer Mischung von Polyacrylat und Polyvinylacetat |
| 30 g/l | Magnesiumchlorid-Hexahydrat |

Neben einem vollen bis leicht steifen Warengriff werden folgende technologischen Effekte erzielt:

| | |
|---|---|
| Naßknitterwinkel (K+S) | 210° |
| Trockenknitterwinkel (K+S) | 198° |
| Monsanto-Bild, md, nach 1 x 20 min 60° C-Wäsche | 3,75–4 |
| Reißfestigkeit, Schuß | 360 N |
| Waschkrumpf nach 1 x 20 min 60° C-Wäsche, | |
| Kette | 0,0% |
| Schuß | 0,5% |
| Formaldehydwerte nach LAW 112-1973 | 0 ppm |

## Beispiel F

Es wird ein Bad (a) mit einem pH-Wert von ca. 4,2 hergestellt durch Mischen folgender Bestandteile: 10% der 50gewichtsprozentigen Lösung von Beispiel 1, 0,2% mit 6 Mol Ethylenoxid umgesetztes i-Octylphenol, 1% einer 25%igen wäßrigen Emulsion eines nichtionogenen Polyethylenweichmachers, 3% einer 45%igen wäßrigen Polyvinylacetatemulsion, 2% einer 25%igen wäßrigen Zinknitratlösung, Rest Wasser.

Mit der angegebenen Lösung werden verschiedene Arten Gewebe auf ca. 55% Flottenaufnahme foulardiert. Die Lösung wird aufgebracht auf gebleichtes, weißes Polyester-/Baumwoll-Bettuchgewebe (50 : 50, 108 g/m²) und auf mit Dispersions-/Küpenfarbstoffen königsblau gefärbtes Polyester-/Baumwollköpergewebe (202 g/m²).

Bei konventioneller Arbeitsweise (precure) wird das foulardierte Gewebe jeweils 1 Minute bei 107° C getrocknet und dann 1½ min bei 150° C kondensiert. Bei einem Teil der Proben werden in die getrocknete (sensibilisierte) Ware Falten eingelegt und darauf wird nach dem post-cure-Verfahren 8 – 5 min bei 120 – 163° C im Ofen behandelt wie bei der Herstellung von permanent-press-Hosen in der Bekleidungsindustrie. Die behandelte Ware ist weitgehend frei von unangenehmem Geruch, und Weißgrad bzw. Färbungen zeigen keine Beeinträchtigung durch die Behandlung.

Bei einem Kontrollversuch (b) werden verwendet: 10% einer 50%igen wäßrigen Dimethylolglyoxalmonoureinlösung und 2,5% einer 25%igen wäßrigen Zinknitratlösung, die übrigen Zutaten wie im Versuch (a).

Proben der nach der konventionellen Arbeitsweise ausgerüsteten Gewebe werden 5mal unter Verwendung eines Waschmittels in einem Haushaltswaschautomaten gewaschen und dann in einem Tumbler getrocknet. Die Eigenschaften sind in der folgenden Tabelle aufgeführt:

| Ware | Behandlung | Nach 5 Haushaltswäschen | |
|---|---|---|---|
| | | Durable Press-Note AATCC Prüfmethode 125-1975 | % Krumpfung (Kette × Schuß) AATCC Prüfmethode 135-1973 |
| 50/50 Polyester-/Baumwoll-Bettuchgewebe | (a) | 4,0 | 1,1 × 0,6 |
| | (b) | 4,0 | 0,9 × 0,8 |
| 65/35 Polyester-/Baumwoll-Bettuchgewebe | (a) | 3,9 | 1,9 × 0,9 |
| | (b) | 3,8 | 2,0 × 0,8 |

Aus den obigen Angaben ergibt sich, daß das erfindungsgemäß erhaltene Produkt bezüglich Permanenz der Bügelfalten und Formtreue der damit ausgerüsteten Textilien ähnliche Eigenschaften hat wie herkömmliche Dimethylolglyoxalmonoureinvernetzer.

Bei der Herstellung von bügelfreien Kleidungsstücken nach dem post-cure-Verfahren kann man das sensibilisierte Gewebe verpackt längere Zeit lagern, bevor man konfektioniert und in der Bügelpresse das Kleidungsstück in der endgültigen Form fixiert. Das mit dem Produkt nach der Erfindung behandelte Gewebe (a) wird unter den für das post-cure-Verfahren üblichen Bedingungen hinsichtlich Temperatur und Zeit behandelt:

| Ware | Probe Nr. | Falten in Bügelpresse fixiert und post-cured bei | |
|---|---|---|---|
| | | Temperatur (C°) | Zeit (min) |
| 65/35 Polyester-/Baumwollkörper, königsblau gefärbt | 1 | 120 | 15 |
| | 2 | 127 | 15 |
| | 3 | 135 | 15 |
| | 4 | 150 | 15 |
| | 5 | 150 | 8 |
| | 6 | 163 | 8 |

Die in dieser Weise pflegeleicht ausgerüsteten Kleidungsstücke werden gewaschen und nach der AATCC-Prüfmethode 88C-1975 geprüft. Die Methode ist ausgelegt auf die Untersuchung der Permanenz von eingebügelten Falten in wash-and-wear-Artikeln. Die Methode ist auch auf die Untersuchung von Falten in Fertigkleidern anwendbar.

| Probe Nr. | Nach 5 Haushaltswäschen | |
|---|---|---|
| | Bügelfalten-Note*) | % Krumpfung (Kette × Schuß) |
| 1 | 4,2 | 2,3 × 1,1 |
| 2 | 4,2 | 2,35 × 1,1 |
| 3 | 4,1 | 2,6 × 1,0 |
| 4 | 4,3 | 2,7 × 1,15 |
| 5 | 4,2 | 2,4 × 1,05 |
| 6 | 4,0 | 2,4 × 1,1 |

*) Benotung: 5 = gut, 1 = schlecht

Die Eigenschaften des nach dem beschriebenen post-cure-Verfahren ausgerüsteten Gewebes sind über einen weiten Vernetzungstemperaturbereich gut. Die hohe Reaktionsfreudigkeit des Produktes gestattet eine Behandlung der Faser unter sehr gemäßigten Bedingungen, so daß ein Abbau der Zellulose vermieden wird.

Weitere textile Werte der mit den Bädern (a) und (b) behandelten post-cure-Gewebe sind in nachstehender Tabelle aufgeführt:

| | Behandlung (a) | Behandlung (b) |
|---|---|---|
| Schweißechtheit – Farbtonänderung | 4,5 | 4,5 |
| – Anbluten | 5 | 5 |
| (AATCC Prüfmethode 15-1976) | | |
| Waschechtheit (70°C) – Farbtonänderung | 4,25 | 4,5 |
| – Anbluten | 3,0 | 3,5 |
| (AATCC Prüfmethode 61-1975) | | |
| Reibechtheit – trocken | 4,5 | 4,5 |
| – naß | 3,75 | 3,5 |
| (AATCC Prüfmethode 116-1977) | | |
| Lichtechtheit – Lichtbogen 40 Stunden | 3,5 | 3,75 |
| (AATCC Prüfmethode 16A-1977) | | |
| Abgasechtheit (AATCC Prüfmethode 23-1975) | 4 | 4 |
| Ozonechtheit (AATCC Prüfmethode 159-1975) | 4 | 4 |
| Pill-Festigkeit (ASTM-D1375-1973) | 4 | 4 |
| Vernähbarkeit | gut | gut |
| Reißfestigkeit – Kette | 946 N | 950 N |
| – Schuß | 559 N | 426 N |
| (ASTM Prüfmethode D1682-84-1975) | | |
| Einreißfestigkeit – Kette | 58 N | 58 N |
| – Schuß | 44 N | 43 N |
| (ASTM Prüfmethode D2261-71) | | |
| Formaldehydgehalt | 0 ppm | 710 ppm |
| (AATCC Prüfmethode 112-1975) | | |

Es ist klar zu erkennen, daß die Ergebnisse der Behandlungen (a) und (b) gleichwertig sind mit der sehr wesentlichen Ausnahme, daß sich das nach (a) behandelte, im Gegensatz zu dem nach (b) behandelten Gewebe als formaldehydfrei erweist.

Beispiel G

Die in Beispiel A erwähnte Baumwollware wird mit folgender Lösung mittels Foulard imprägniert (Flottenaufnahme ca. 70%).

200 g/l der nach Beispiel 4 hergestellten 50%igen Vernetzerlösung
30 g/l Magnesiumchlorid-Hexahydrat

und bei 110°C auf eine Restfeuchte von ca. 8% getrocknet. Anschließend wird 4 min bei 150°C

kondensiert. Das auf diese Weise behandelte Gewebe liefert folgende Testergebnisse:

| | |
|---|---|
| Naßknitterwinkel (K+S) | 195° |
| Trockenknitterwinkel (K+S) | 204° |
| Monsanto-Bild, md, nach 1 × 20 min 60° C-Wäsche | 3,75 |
| Reißfestigkeit, Schuß | 25,2 Kp |
| Waschkrumpf nach 1 × 60° C-Wäsche, | |
| Kette | 1,0% |
| Schuß | 1,0% |
| Freie Formaldehydwerte nach LAW 112-1973 | 0 ppm |

### Beispiel H

Ein Baumwollnesselgewebe (siehe Beispiel A) wird mit einer wäßrigen Lösung von 200 g/l der nach Beispiel 6 hergestellten 45%igen Vernetzerlösung und 45 g/l Magnesiumchlorid-Hexahydrat als Katalysator imprägniert, auf eine Flottenaufnahme von ca. 70% abgequetscht und bei 110° C auf eine Restfeuchte von 7–8% getrocknet. Anschließend wird 5 min bei 145° C kondensiert. Folgende Testergebnisse werden erhalten:

| | |
|---|---|
| Naßknitterwinkel (K+S) | 196° |
| Trockenknitterwinkel (K+S) | 176° |
| Monsanto-Bild, md, nach 1 × 20 min | 3,5 |
| Reißfestigkeit, Schuß | 350 N |
| Waschkrumpf nach 1 × 60° C-Wäsche, | |
| Kette | 1,2 |
| Schuß | 0,8 |

### Vergleichsversuch 1

Die in Beispiel A erwähnte Baumwollware wird mittels Foulard bei einer Flottenaufnahme von ca. 70% mit folgender Ausrüstungsflotte imprägniert:

120 g/l einer 50%igen wäßrigen Lösung von N,N'-Dimethylol-4,5-dihydroxy-äthylenharnstoff
18 g/l Magnesiumchlorid-Hexahydrat

Trocknung und Kondensation gemäß Beispiel 7.
Folgende technologischen Werte werden gemessen:

| | |
|---|---|
| Naßknitterwinkel (K+S) | 246° |
| Trockenknitterwinkel (K+S) | 275° |
| Monsanto-Bild, md, nach 60° C-Wäsche | 4,75 |
| Reißfestigkeit, Schuß | 274 N |
| Waschkrumpf, nach 1 × 60° C-Wäsche, | |
| Kette | 0,0% |
| Schuß | 0,2% |
| Formaldehydwerte nach LAW 112-1973 | 650 ppm |

### Vergleichsversuch 2

Die in Beispiel A erwähnte Baumwollware wird mittels Foulard bei einer Flottenaufnahme von ca. 70% mit folgender Ausrüstungsflotte imprägniert:

150 g/l N,N'-Dimethyl-4,5-dihydroxy-äthylenharnstoff
30 g/l Magnesiumchlorid-Hexahydrat

Trocknung und Kondensation gemäß Beispiel A.
Folgende technologischen Werte werden gemessen:

| | |
|---|---|
| Naßknitterwinkel (K+S) | 176° |
| Trockenknitterwinkel (K+S) | 168° |
| Monsanto-Bild, md, nach 60° C-Wäsche | 3,0 |
| Reißfestigkeit, Schuß | 396 N |

| | |
|---|---|
| Waschkrumpf, nach 1 × 60° C-Wäsche, | |
| Kette | 1,5% |
| Schuß | 0,8% |
| Formaldehydwerte nach LAW 112-1973 | 0 ppm |

Auf der Ware ist eine relativ starke Vergilbung festzustellen.

Vergleichsversuch 3

Die in Beispiel 7 erwähnte Baumwollware wird mittels Foulard bei einer Flottenaufnahme von ca. 70% mit folgender Ausrüstungsflotte imprägniert:

| | |
|---|---|
| 150 g/l | N,N'-Dimethyl-4,5-dimethoxy-äthylenharnstoff, ungepuffert |
| 30 g/l | Magnesiumchlorid-Hexahydrat |

Trocknung und Kondensation gemäß Beispiel A.
Folgende technologischen Effekte werden gemessen:

| | |
|---|---|
| Naßknitterwinkel (K + S) | 123° |
| Trockenknitterwinkel (K + S) | 90° |
| Monsanto-Bild, md, nach 1 × 60° C-Wäsche | 2 |
| Reißfestigkeit, Schuß | 400 N |
| Waschkrumpf, nach 1 × 60° C-Wäsche, | |
| Kette | 4,0% |
| Schuß | 3,0% |
| Formaldehydwerte nach LAW 112-1973 | 0 ppm |

Es ist eine deutliche Vergilbung sowie ein unangenehmer Geruch der Ware wahrzunehmen.

**Patentansprüche**

1. Verfahren zur Herstellung von lagerbeständigen, formaldehydfreien Pflegeleicht-Ausrüstungsmitteln für cellulosehaltige Textilien auf der Basis von Harnstoff oder N,N'-disubstituierten Harnstoffen und Glyoxal, dadurch gekennzeichnet, daß man in einer ersten Verfahrensstufe 1 bis 1,1 Mol Harnstoff oder einen symmetrisch disubstituierten Harnstoff der allgemeinen Formel I

$$R^1-HNCONH-R^2 \qquad I$$

in der $R^1$ und $R^2$ ein Wasserstoffatom, einen Alkylrest mit 1 bis 4 C-Atomen, Hydroxyethyl, Methoxyethyl oder Methoxypropyl bedeuten, mindestens drei Stunden in wäßriger Lösung zusammen mit einem Mol Glyoxal in Gegenwart von Puffergemischen bei pH-Werten zwischen 4 und 6,8 und Temperaturen von 20 bis 60° C hält und die erhaltene Lösung in einer zweiten Stufe mit mindestens 0,5 Mol eines Alkohols $R^4OH$, wobei $R^4$ für Methyl, Ethyl, Methoxyethyl, Hydroxyethyl, Methoxyethoxyethyl oder für den Rest eines Polyethylenglykols mit bis zu 10 Ethylenoxideinheiten steht, zu einer Verbindung der allgemeinen Formel II

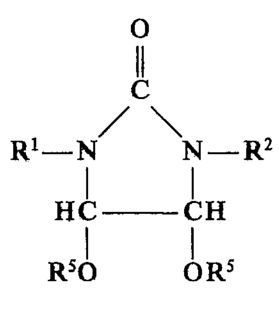

(II)

in der $R^1$ und $R^2$ die für Formel I angegebenen Bedeutungen haben und $R^5$ Wasserstoffatome bedeuten, die zu mindestens 25% durch einen Rest $R^4$ mit den obengenannten Bedeutungen ersetzt sind, umsetzt, indem man unter Zusatz einer Säure 1 bis 4 Stunden bei pH-Werten zwischen 2 und 4,5 und Temperaturen von 30 bis 65° C hält und schließlich den pH-Wert auf 4 bis 7 einstellt.

2. Verfahren zur formaldehydfreien Pflegeleichtausrüstung von cellulosehaltigen oder daraus bestehenden Textilien durch Imprägnieren derselben mit einer wäßrigen Lösung, enthaltend 2,5 bis 10

Gewichtsprozent, gerechnet als Festsubstanz, eines formaldehydfreien, vernetzbaren Ausrüstungsmittels und 0,6 bis 4 Gewichtsprozent eines sauren oder potentiell sauren Katalysators und Fixieren durch Erhitzen für 10 sec bis 15 min auf 100 bis 230°C, wobei das gemäß Anspruch 1 erhaltene Ausrüstungsmittel eingesetzt wird.

**Claims**

1. A process for the production of storage-stable, formaldehyde-free, easy-care finishing agents for cellulosic textiles on the basis of urea or N,N'-disubstituted ureas and glyoxal, wherein, in the first stage of the process, 1 to 1.1 moles of urea or symmetrically disubstituted urea of the general formula I

$R^1-HNCONH-R^2$ I

where $R^1$ and $R^2$ are hydrogen, alkyl of 1 to 4 carbon atoms, hydroxyethyl, methoxyethyl or methoxypropyl, are kept for at least threee hours in aqueous solution with 1 mole of glyoxal, in the presence of buffer mixtures which maintain the pH of the solution between 4 and 6.8, at a temperatur of from 20 to 60° C, and, in the second stage, the solution resulting from the first stage is reacted with at least 0.5 mole of an alcohol $R^4OH$, where $R^4$ is methyl, ethyl, emthoxyethyl, hydroxyethyl, methoxyethoxyethyl or the radical of a polyethylene glycol containing up to 10 ethylene oxide units, in the presence of an acid for 1 to 4 hours at a pH of from 2 to 4.5 and a temperature of from 30 to 65° C the pH of the solution being finally adjusted to 4 to 7, to give a compound of the general formula II

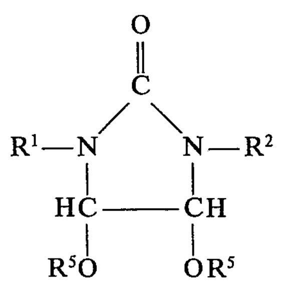

(II)

where $R^1$ and $R^2$ have the meanings given for formula I, and the $R^5$'s are each hydrogen, at least 25% of which is replaced by a radical $R^4$ having the above meanings.

2. A process for the formaldehyde-free, easy-care finishing of textiles containing or consisting of cellulose by impregnating same with an aqueous solution containing 2.5 to 10% by weight, calculated as solid, of a formaldehyde-free crosslinkable finishing agent and 0.6 to 4% by weight of an acid or potentially acid catalyst and fixing by heating for from 10 seconds to 15 minutes at from 100 to 230° C, wherein the finishing agent obtained according to claim 1 is used.

**Revendications**

1. Procédé de préparation d'apprêts exempts de formaldéhyde et stables pour matières textiles contenant de la cellulose, faciles à entretenir, à base d'urée ou d'urées, N,N'-disubstituées et de glyoxal, caractérisé en ce que, dans un premier stade opératoire, on maintient pendant au moins trois heures en solution aqueuse, à des températures de 20 à 60° C et en présence de mélanges tampons réglant le pH entre 4 et 6,8, 1 à 1,1 mole d'urée ou d'une urée disubstituée symétrique de la formule générale I

$R^1-HNCONH-R^2$ (I)

dans laquelle $R^1$ et $R^2$ désignent chacun un atome d'hydrogène, un radical alcoyle en $C_1$ à $C_4$ ou un groupe hydroxy-éthyle, méthoxy-éthyle ou méthoxy-propyle, et 1 mole de glyoxal, puis, dans un deuxième stade, on fait réagir la solution obtenue avec au moins 0,5 mole d'un alcool $R^4OH$, où $R^4$ désigne un groupe méthyle, éthyle, méthoxy-éthyle, hydroxy-éthyle ou méthoxy-éthoxy-éthyle ou le reste d'un poly-éthylène-glycol comportant jusqu'à 10 unités oxyde d'éthylène, pour obtenir une substance de la formule générale II

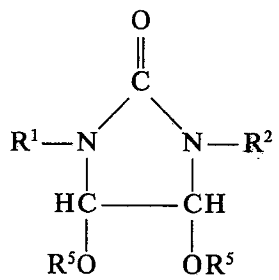

(II)

dans laquelle $R^1$ et $R^2$ possèdent les significations définies pour la formule I et chaque $R^5$ désigne un atome d'hydrogène, au moins 25% de ceux-ci étant remplacés par un groupe $R^4$ possédant les significations définies plus haut, en maintenant le mélange réactionnel pendant une à quatre heures à des températures de 30 à 65° C, le pH étant ajusté par addition d'un acide entre 2 et 4,5, et on ajuste finalement à un pH entre 4 et 7.

2. Procédé pour un apprêtage exempt de formaldéhyde, facile à entretenir, de matières textiles cellulosiques ou contenant de la cellulose par imprégnation de ces matières textiles avec une solution aqueuse d'une teneur de 2,5 à 10% en poids, calculés en tant que substance solide, d'un agent d'apprêtage réticulable, exempt de formaldéhyde, et de 0,6 à 4% en poids d'un catalyseur acide ou potentiellement acide et fixation par un chauffage de 10 secondes à 15 minutes entre 100 et 230° C, l'agent d'apprêtage étant un des apprêts obtenus par le procédé selon la revendication 1.